# EUROPEAN PATENT APPLICATION

(11) **EP 2 510 877 A1**
(43) Date of publication of application: **17.10.2012**
(21) Application number: 11162648.7
(22) Date of filing: 15.04.2011
(51) Int. Cl.: A61B 5/20, A61F 5/453, G01N 33/493

(54) **Analysing device for urine**

(71) Applicant: Redoak S.r.l., 20123 Milano (MI) (IT)
(72) Inventor: Longoni, Giovanni, 20030, Bovisio Masciago (Milano) (IT); Orsenigo, Renzo, 20048, Carate Brianza (Milano) (IT)
(74) Representative: Lunati, Valerio

(57) **Abstract**

It is provided an analysing device (1) for urine adapted to determine the value of physico-chemical features of an urine sample exactly as it is; said analysis device (1) comprising a fluid admission system including a sampler (60a) adapted to be brought into connection for fluid passage with the patient and to measure an urine portion to be analysed; an analysis station (20) put in connection for fluid passage with the fluid admission system and adapted to carry out the analysis of the urine to be analysed and comprising a plurality of ionoselective electrodes (22), each of which is suitable to determine at least one first parameter that is a function of at least one of the values of the physico-chemical features, and wherein the command and control unit (70) is adapted to carry out an interpolation between the first parameters for determining at least one of the values of the physico-chemical features.

## Description

The present invention relates to an analysing device for urine of the type pointed out in the preamble of the first claim.

The device, in accordance with the invention, can be placed in the vicinity of a bed, at least partly under said bed for example, and can be connected directly to a patient, through a catheter. In detail, the device is adapted to carry out an analysis of urine or urinalysis of the electrochemical type.

It is known that presently urinalysis is one of the main methods of analysis to demonstrate or exclude many pathologies/problems such as structural lesions, the presence of infectious agents, an alteration of one or more organs. In particular, urinalysis is the main form of analysis for kidneys and therefore it is an important test enabling to recognise possible kidney malfunctions and, as a result, the state of the kidneys themselves.

One of the main types of analyses is the electrochemical one in which the potential difference between a pair of electrodes is calculated and, based thereon, the contents of pH, sodium, potassium, ammonium and chloride in urine are determined. This analysis is presently executed as hereinafter described.

Connected to the patient is a catheter enabling urine to be conveyed into a suitable container, such as a bag, from which the medical staff takes the correct urine amount. This drawing can take place either through a syringe or other similar instruments suitably introduced into the bag, or through a suitable dropper adapted to measure the correct urine amount.

Then the amount taken from the bag is diluted so as to reduce the interference with disturbing substances that can lead to incorrect evaluations of the sample and, consequently, to a wrong analysis.

Once the urine sample has been diluted, it is introduced into the analysing device that, through a given process, carries out the analysis or test.

In detail, reactants and/or chemical solutions tending to cancel the negative influence given by the presence of disturbing substances that can give rise to an incorrect analysis are added to the urine sample. These added elements vary based on the analysis to be performed and for instance can be the urease enzyme used for urea analysis and the cation exchange column necessary for removing/reducing the high content of ammonium ions.

Once the sample is ready for being analysed, it is for instance disposed between two electrodes and by measuring the potential difference between said electrodes, it is determined the content of the sought substance in carrying out the analysis.

The technical art mentioned above however has some important drawbacks.

In fact, the process used is particularly long and asks for the continuous presence of medical staff. In particular, drawing of the urine amount to be analysed, carried out by the operator may cause contamination of the sample, in addition to an obvious increase in the test time.

Another problem is represented by the analysis itself that is particularly complicated and of difficult accomplishment.

In particular, since the urine content is very varying both from patient to patient and between different samples taken from the same patient, a different dilution with reactants is required each time.

Another critical aspect resides in the fact that the analysis is also a function of temperature, as temperature affects the final result in a decisive manner.

The above aspects therefore determine a difficult adjustment of all the parameters and therefore lead to a non-optimal analysis of the urine sample. Under this situation, the technical task underlying the present invention is to conceive an analysing device for urine capable of substantially obviating the mentioned drawbacks.

Within the scope of this technical task it is an important aim of the invention to conceive a device enabling simple, quick and frequent analyses involving a substantial monitoring of the patient's conditions.

In fact, the fundamental organs for survival are the heart, lungs and kidneys and, while there are different machines adapted to monitor heart and lungs through analysis of the respiratory and cardio-circulatory systems, kidneys cannot be monitored by known machines.

A further aim of the invention is to make available a device that does not ask for the presence of medical and paramedical staff and therefore a device enabling analyses to be carried out in a substantially automatic manner.

The technical task mentioned and the aims specified are achieved by an analysing device for urine as claimed in the appended Claim 1.

Preferred embodiments are highlighted in the sub-claims.

The features and advantages of the invention are hereinafter clarified by the detailed description of a preferred embodiment of the invention, with reference to the accompanying drawings, in which:

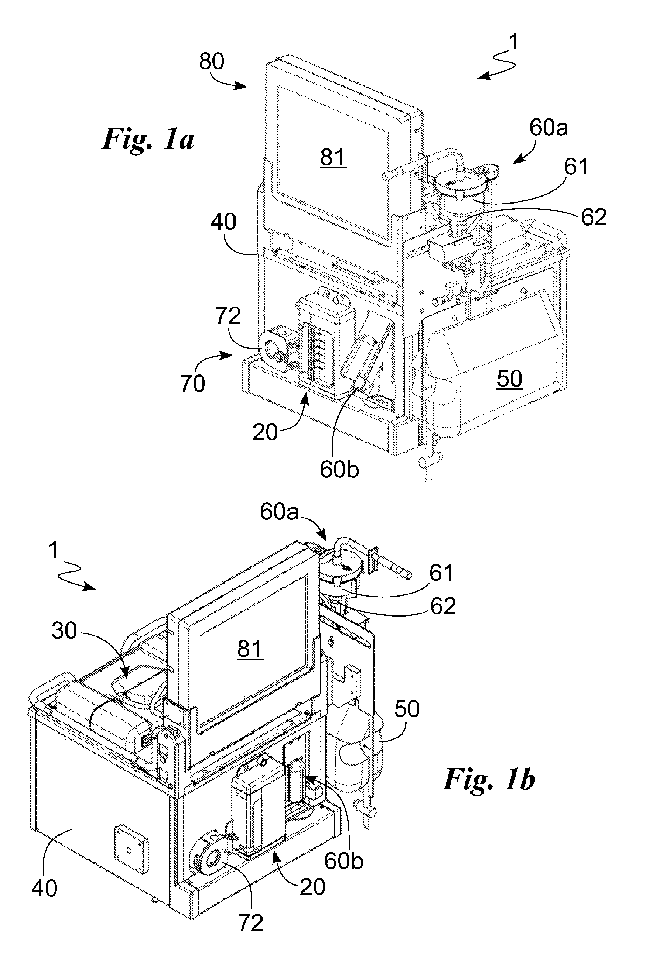

**Fig. 1a** shows a view of the analysing device according to the invention;
**Fig. 1b** is a second view of the analysing device;
**Fig. 2** shows an assembly of the analysing device;
**Fig. 3** shows a section of a second assembly of the analysing device;
**Fig. 4** represents a third assembly of the device seen in section;
**Fig. 5** diagrammatically shows the connections for fluid passage belonging to the analysing device of the invention; and
**Fig. 6** is a model of an analysis carried out by the device.

With reference to the drawings, the analysing device for urine according to the invention is generally identified by reference numeral **1**.

In particular, it is adapted to be used for analysing an urine sample, preferably exactly as it is (i.e. without any addition of reactants or solutions), determining the value of physico-chemical features thereof and, more specifically, device 1 determines the pH and the sodium, potassium, ammonium and chloride contents of an urine sample and, simultaneously, determines the instantaneous urinary flow thereof.

The analysing device 1 shortly comprises a holding structure adapted to contain at least part of the components of device 1, an admission system adapted to enable introduction of the fluid to be analysed into device 1; an analysis or test station **20** placed in connection for fluid passage with the admission system and adapted to carry out said analysis and to analyse at least part of said portion of fluid to be analysed; a calibrating/washing block **30** containing calibrating and washing substances/liquids; a holding structure **40** adapted to support the components of device 1 in a correct position; and a discharge tank **50** adapted to collect the fluid to be analysed that has been rejected, i.e. not used for the analysis process.

Preferably, the holding structure has such sizes that it can be easily disposed under the patient's bed and can be provided with handling means such as swivelling wheels for example, adapted to facilitate movement of the analysing device 1.

Said admission system can consist of a sampler **60a** adapted to be brought into connection with the patient for fluid passage, enabling urine to reach the device exactly as it comes out of the patient and adapted to meter the fluid entering the device. Alternatively, urine can come from a known admission member **60b** adapted to allow an operator to introduce a sample of fluid previously taken from the patient into the device. To said sample preferably reactants or other solutions have not been added. Preferably, the analysing device 1 has both the sampler 60a and the admission member 60b.

Sampler 60a comprises a collecting tank **61** adapted to collect the fluid coming from the patient; a dripping device **62** of known type, adapted to divide the fluid coming from the collecting tank 61 into drops; a continuous-signal optical counter **63** adapted to count the drops coming out of the dripping device 62; a collecting trap **64** placed downstream of the dripping device 62 and adapted to collect a predetermined amount of fluid metering a predetermined portion of same; and an overflow exhaust duct **65,** i.e. a duct adapted to drain the possible excess flow into the discharge tank 50.

Tank 61, in order to avoid a contamination by external agents, has a collecting cavity **61a** suitably separated from the outside by a lid **61 b** to which a catheter or other similar element can be fastened for bringing the connecting cavity 61 a into connection with the patient for fluid passage.

The lower part of tank 61, i.e. the part opposite to lid 61 b and therefore to cavity 61 a is in the form of a truncated cone or a truncated pyramid in section so as to promote entry of fluid into the dripping device 62 thus avoiding residues in the chamber itself.

Below the dripping device 62 the sampler contemplates the presence of the continuous-signal optical counter 63 that by counting the number of drops going from tank 61 to trap 64, quantifies the patient's fluid flow.

The optical counter 63 substantially has two distinct components essentially placed on opposite sides relative to the drop coming out of the dripping device 63 and, more specifically, placed in mirror image relationship relative to the drop. In detail, it includes an optical emitter **63a**, a photodiode for example, adapted to emit a light signal, a photo-sensitive element **63b,** such as a phototransistor, adapted to pick up the presence/absence of the light signal and suitably connected to a command and control unit **70** that, based on the received signal, determines the number of drops, as better explained hereinafter. Preferably, the optical emitter 63a is adapted to emit a continuous signal and, more preferably, a constant continuous signal, i.e. characterised by a substantially constant intensity.

As an alternative to the preceding arrangement, the optical counter 63 can have the emitter 63a and element 63b that are not in mirror image relationship relative to the drop coming out of the dripping device 63 and, in this case, it can be provided with reflecting means, such as mirrors, adapted to direct the light signal against the photo-sensitive element 63b.

Once the drop has passed through the optical counter 63, it enters the trap comprising a first duct **64a** having an S-shaped extension line.

In detail, in the first duct 64a a first U-shaped portion and a second portion in the form of an inverted "U" can be identified; the first U-shaped portion in which a predetermined fluid amount is collected, is brought into connection for fluid passage with the dripping device 63 while the second portion is in connection for fluid passage with the discharge tank 50, through the overflow exhaust duct 65 for example. More specifically, the lower part of the first portion, i.e. that in which said fluid amount is collected, is in connection for fluid passage with the control station through a suitable pipe not shown in the figure.

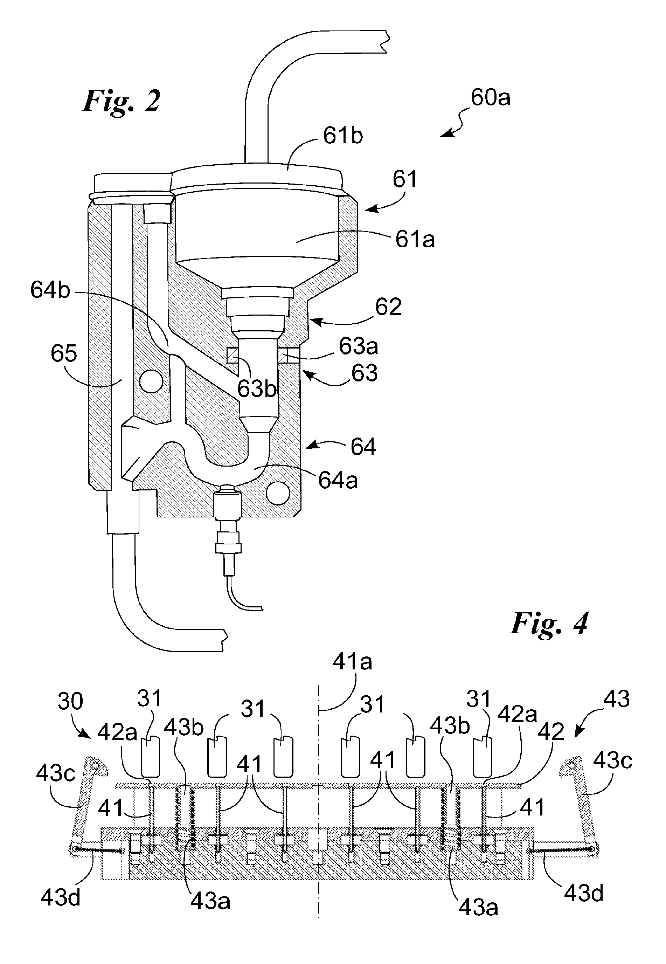

The collecting trap 64 also includes a second duct **64b** adapted to put two sections of the first portion in connection for air passage both with each other and with the external environment. In particular, the second duct 64b substantially has a Y-shaped conformation with an end connected to the outside, under fluid passage conditions, and the two other ends linked to the first duct 64a on the opposite side relative to said pipe, as shown in Fig. 2.

The fluid, once calculated by counter 63, comes out of trap 64 and is guided to the analysis station 20 in which fluid analyses are carried out.

This analysis station 20 is adapted to perform an electrochemical analysis of the fluid and has a plurality of electrodes each of which is adapted to determine at least one first parameter, a potential difference for example, for each of the physico-chemical features. If the analysis concerns urine, the analysis station 20 may comprise at least five electrodes with possibility of further extension and, more exactly, a reference electrode **21** and at least one ionoselective electrode **22** or other similar element adapted to interact with the substance to be analysed.

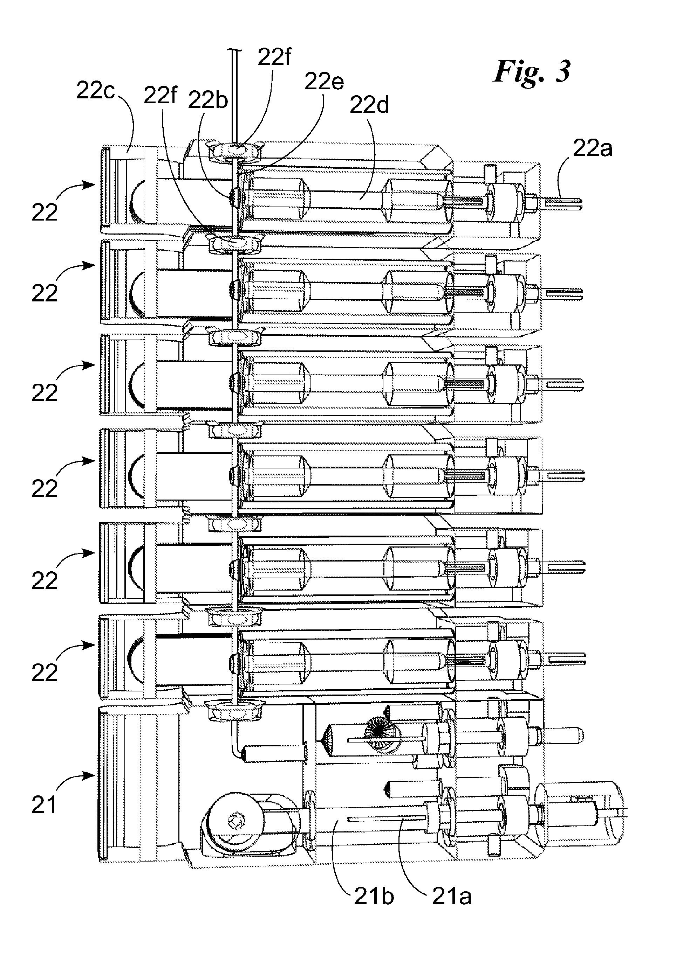

In detail, at least one ionoselective electrode 22 is provided for each of the following features to be analysed: pH, sodium, potassium, ammonium and chloride contents. For instance, in Fig. 3 an analysis station is shown that is provided with six ionoselective electrodes 22, i.e. one for pH analysis, four for detecting the sodium, potassium, ammonium and chloride contents, while the sixth one not used for this particular analysis can be subsequently used for detecting a further physico-chemical feature of an urine sample.

The reference electrode 21 briefly has a conductive element **21a**, of silver for example, housed in a chamber **21b** containing a fluid adapted to keep the electrode 21 to an always constant potential.

Each ionoselective electrode 22 is in contact with the fluid and is adapted to react with a given ion. In detail, the ionoselective electrode 22 has an electric connector **22a** adapted to enable the ionoselective electrode 22 to be brought into electric connection with the standard one 21 in the presence of the fluid to be analysed; a ionosensitive membrane **22b** or other similar element adapted to react with a given ion; a central body **22c** adapted to contain almost the whole of the elements constituting the ionoselective electrode 22 and defining two distinct chambers: a connecting chamber **22d** interposed between membrane 22b and connector 22a and adapted to contain a fluid characterising the offset value of the electrodes, a saturated KCl solution for example, and a holding chamber **22e** adjacent to the ionoselective electrode 22 and adapted to contain urine.

In particular, the holding chamber 22e preferably consists of a through hole so that, once the ionoselective electrodes 22 are positioned, the holding chambers 22e form a single duct in such a manner as to enable a free fluid passage to exist both between the chambers themselves and at the exit/entry of the analysis station 20. In order to avoid fluid leakage, sealing elements can be interposed between said electrodes, such as O-rings **22f,** for example.

Each of the ionosensitive electrodes 22 is adapted to react with only one of the substances to be analysed. In detail, in case of an analysing device 1 adapted to determine the pH and the sodium, potassium, ammonium and chloride contents, the ionoselective electrodes 22 are sensitive to at least one of the following ions H⁺, Na⁺, K⁺, NH₄⁺ and Cl⁻.

This selection is carried out by means of ionosensitive membranes 22b suitably made and optimised for the element with which they have to interact. For instance membranes for H⁺ and Na⁺ ions have a glass matrix while membranes for K⁺, NH₄+ and Cl⁻ have a polymeric matrix with a high molecular weight.

The analysing device 1 at the analysis station 20 has a thermostat **23** adapted to adjust the temperature of the fluid to be analysed and of the calibrating element when the fluid is in the analysis station 20. This thermostat consists of any element, an electric resistor for example, adapted to maintain the fluid temperature substantially constant. Preferably, this temperature is approximately included between 30°C and 45°C and more preferably is substantially equal to 37°C.

Each electrode 21 and 22 is finally electrically connected to the command and control unit 70 adapted to analyse data collected by each ionoselective electrode 21 and compare said collected data with those related to the standard electrode 21, and to operate the calibrating/washing block 30 necessary for preparation of the analysis station 20 before carrying out an analysis.

The command and control unit 70 in addition to analysing data from the analysis station 20 is preferably connected to sampler 60a and, more exactly, to the optical counter 63 so as to quantify the fluid flow to be analysed and entering device 1.

The calibrating/washing block 30 comprises a plurality of vats **31** that can be suitably connected to the analysis station 20 and a support **42** adapted to contain said vats 31 enabling all vats 31 either to be simultaneously moved or to be moved in an independent manner. In detail, vats 31 are preferably fastened to the support by releasable connecting means, such as friction couplings.

The calibrating/washing block 30 preferably has six vats 31 three of which are adapted to contain the washing fluid and the maximum and minimum calibrating elements before their use, and three adapted to contain the waste from the analysis or calibration process.

In particular, the maximum and minimum calibrating elements consist of fluids having known physico-chemical features to be analysed and high and low contents respectively of the same physico-chemical features.

Said high and low contents are preferably close to the maximum and minimum contents respectively relative to the supposed values obtainable by the analysis.

More specifically, the maximum calibrating element consists of a fluid having a pH and known sodium, potassium, ammonium and chloride contents preferably greater than the maximum value that can be found in a urine sample, while the minimum calibrating element consists of a fluid having a pH and known sodium, potassium, ammonium and chloride contents preferably smaller than the minimum value that can be found in a urine sample.

Vats 31 consist of known medical bags provided with suitable connecting means adapted to enable easy insertion and removal of a needle for example, without the occurrence of undesirable liquid escape from the vat. Insertion/removal is obtained by introducing/withdrawing the calibrating/washing block 30 into/from the holding structure 40. In detail, block 30 is inserted in register with suitable insertion means **41**, needles for example, belonging to the holding structure 40.

This operation, better described in the following, is obtained due to the presence of a particular lifting mechanism adapted to enable needles 41 to be introduced into vats 31 when the block 30 is inserted in structure 40 and to be removed when block 30 is withdrawn from structure 40.

The lifting mechanism comprises a plate **42** adapted to be disposed between structure 40 and block 30 and provided with holes **42a** formed at the insertion means 41; and an actuating system **43** adapted to enable plate 42 to be moved along the extension direction **41a** of the insertion means 41.

In detail, the actuating apparatus 43 is of the passive type, i.e. it is adapted to be moved by exerting an external force, on block 30 for example, and therefore is preferably devoid of motors or other similar elements adapted to move it irrespective of an external action. This apparatus 43 can therefore have one or more springs **43a** for maintaining plate 42 at least partly over the insertion means 41 preventing said means from appearing beyond the plate itself, and guide elements **43b**, guides for example, enabling plate 42 to move downwards along the extension direction 41a making the insertion means 41 emerge beyond said plate 42 and therefore causing insertion of said means 41 into vats 31.

The actuating system 43 can finally contemplate levers **43c** or other similar elements adapted to prevent the insertion means 71 from coming out of plate 42 in the absence of the calibrating/washing block 30 and provided with suitable spring means **43d** adapted to operate motion of said levers 43c in at least one direction.

Levers 43c are hinged on structure 40 so that they can rotate relative to the structure and define a safety position and an introduction position. In particular, in the safety position, i.e. in the absence of block 30, levers 43c are in such a position that they lock plate 42 relative to means 41 so that the plate 42 is allowed to hide said means. On the contrary, in the introduction position, i.e. upon introduction of block 30, the position of levers 43c enables a mutual motion between plate 42 and the insertion means 41 and consequently insertion of means 41 into vats 31.

Alternatively, the lifting mechanism can contemplate the presence of holes 42a on structure 40, plate 42 being provided with the insertion means 41 and being adapted to be placed on the opposite side of block 30, relative to holes 42a. The analysing device 1 further has a command unit put in connection for fluid passage and adapted to control flowing of at least the fluid to be analysed; a governing post 80 adapted to enable operation of device 1; and an export element such as a printer or a mass peripheral connection adapted to enable the results to be printed and/or examined on an outer device such as a computer.

The command unit is preferably included in the command and control unit 70 that therefore comprises a plurality of solenoid valves **71** adapted to control the flow entering and coming out of the analysis station 20; a plurality of ducts adapted to connect at least part of the components of device 1 for fluid passage; and a pump **72** or other similar member adapted to move the fluid to be analysed.

The governing post 80 is adapted to enable almost all the functions of device 1 to be controlled. In fact it allows the urine volume produced by the patient to be monitored through the optical counter 63 so as to establish whether an urine content sufficient for carrying out an analysis is present in the collecting trap 64.

In addition, the governing post 80 allows analyses to be programmed at predetermined hours or, alternatively, analyses to be programmed at predetermined time intervals, so that the regular operation of the device can be checked and consequently correct execution of the analyses without the presence of the operator.

Said governing post 80 comprises a screen **81** o other similar element enabling at least the analysis results to be displayed and control members, such as a keyboard adapted to enable operation of the whole device 1 to be controlled. Alternatively, screen 81 can be a touchscreen and it can also control the analysing device 1 in addition to data displaying.

Finally, the analysing device 1 has a powering system not shown in the figure adapted to supply energy to the components of device 1, such as the command and control unit 70, analysis station 20 and unit 70; said system may consist of a battery and/or a connecting cable for connection of the device to an outer electricity supply.

Operation of an analysing device, described above as regards structure, is the following.

Initially, device 1 is prepared by introducing the calibrating/washing block 30 into the holding structure 40.

In particular, introduction of block 30 causes levers 43c to be set in motion so that plate 42 through translation moves from the starting safety position to the insertion position, thereby enabling appearance of the insertion means 41 that therefore are introduced into vats 31.

More specifically, movement of levers 43c makes the insertion means 41 translate relative to plate 42 along the extension direction 41 so that said means emerges from the plate and can therefore be inserted into vats 31.

When this operation has been completed, the device is ready for use and the fluid sample, typically the urine sample can be introduced for being analysed, through the admission member 60b or, preferably, sampler 60a.

In detail, in case of use of the admission member 60b, the sample is manually inserted by a medical or paramedical operator into said member 60b using a syringe or other vessel adapted to ensure no contamination of the fluid sample to be analysed.

Alternatively, introduction takes place through sampler 60a that, being directly connected to the patient through a catheter, allows the urine to directly reach the inside of the analysing device 1.

In this case, the fluid to be analysed, i.e. urine, reaches the collecting tank 61 and is received inside the collecting cavity 61 a, being then quantified through the optical counter 63. In some cases, if the fluid flow reaching sampler 60a is too much and the level in the collecting tank 61 exceeds a given level, the excess fluid amount, i.e. the amount above said level, is directly ejected by the overflow exhaust duct 65 into tank 50 therefore preventing this excess amount from reaching the dripping device 62 and the optical counter 63.

The fluid present in tank 61, under the action of the force of gravity, passes through the dripping device 62 and is divided into drops that, in turn, cross the optical counter 63 and interrupt the light beam, i.e. the continuous signal emitted by the optical emitter 63a and detected by the photosensitive element 63b.

In detail, each drop, by its interposition between emitter 63a and photosensitive element 63b interrupts the light beam striking element 63b and therefore the command and control unit 70, suitably brought into connection of current/data passage with the photosensitive element 63b, detects interruption of the continuous signal and stops reading of said continuous signal for a predetermined non-reading time.

Once the fluid to be analysed has crossed the optical counter 63, it reaches the collecting trap 64 and, more exactly, the first duct 64a where part of the fluid, after travelling along the whole first duct 64a, reaches the exhaust duct 65 and is therefore discharged into the discharge tank 50; while a second part of fluid, the amount of which depends on the size of the first duct 64a, remains inside the first U-shaped portion of the first duct 64a until said fluid is needed for carrying out the analysis.

In detail, said second part of fluid, corresponding to the amount to be analysed, remains inside the first portion of the first duct 64a thanks to the second duct 64b that, by making two sections of the first duct 64a and the external environment communicate with each other, enables said sections to have the same pressure so that the fluid can remain between the two pressures.

At this point, device 1 is ready for carrying out the analysis of the urine sample. However, before the first use or after a given number of carried out analyses, the device can need a calibration.

To carry out such a calibration, initially the maximum and minimum calibrating elements are analysed, i.e. a full analysis is carried out on a sample of maximum calibrating element and a sample of minimum calibrating element.

In particular, this analysis is performed at a standard temperature, adjusted by thermostat 23 and preferably this temperature is about 37°C.

Therefore, through the governing post 80, a sample of fluid, of the maximum calibrating element for example, is taken from the respective vat 31 and sent to the analysis station 20 and, more specifically, into the duct formed by the holding chambers 22e of the electrochemical sensors 22.

In particular, each ionoselective membrane 22b selects at least one respective ion and, in some cases, together with said respective ion, similar ions. Consequently, each ionoselective electrode 22 reacts with the respective ion and possibly with said similar ions. Based on the ion amount or concentration with which each individual ionoselective electrode 22 interacts, the electrode 22 itself measures a potential difference relative to the reference electrode 21, determining a first parameter.

Said first parameter is a function of the selected ion and of said possible similar ions. Device 1 is therefore adapted to interpret said first parameters and to extract the physico-chemical features of the analysed sample therefrom.

If it is determined that the ionoselective electrodes 22 are adapted to select and measure the respective ions consisting of: H⁺ ions (for measuring the pH), K⁺ ions (for measuring potassium) and Cl⁻ (for measuring chloride), they select and measure the individual respective ions by themselves. Therefore the concentration of these ions is proportional to the potential difference measured by the respective ionoselective electrode 22. Consequently, from the first parameters obtained from the ionoselective electrodes 22 related to H⁺, K⁺ and Cl⁻ it is possible to directly obtain the concentration of the respective ions.

In a different way, the first parameter measured by the ionoselective electrode 22 related to the Na⁺ ion (for measuring sodium) is a function of the concentration of Na⁺ ions and also of H⁺ ions. In particular, the sodium concentration can be drawn from said related first parameter and is dependent on a cubic function of the concentration of the H⁺ ions. (The concentration of H⁺ ions can be in turn directly drawn from the respective ionoselective electrode 22, as previously mentioned).

In addition, the first parameter measured by the ionoselective electrode 22 related to the NH₄⁺ ion (for measuring ammonium) is a function of the concentration of NH₄⁺ ions and also of K⁺ ions. In particular, the ammonium concentration can be drawn from said related first parameter minus a linear function of the concentration of the K⁺ ions. (The concentration of K⁺ ions can in turn be directly drawn from the respective ionoselective electrode 22, as previously mentioned).

In summary, the command and control unit 70 first of all determines said first parameters, at least one of which directly determines at least one of the values of the physico-chemical features to be determined. Subsequently, the same unit 70 determines further physico-chemical features as a function of said first parameters and of the already determined physico-chemical features. Consequently, the command and control unit 70, based on the signals sent to electrodes 21 and 22, quantifies said potential difference and therefore determines the results of the analysis, i.e. in the example in question the pH and the sodium, potassium, ammonium and chloride contents present in the sample.

In conclusion, at the end of the analysis of the maximum sample, the command and control unit 70 determines for each of the physico-chemical features, a maximum pair in which there are indicated the content of a physico-chemical feature and the first parameter relating to the same feature, which are obtained through analysis of the maximum calibrating element. For instance, the maximum pair relating to the pH identifies two distinct values, i.e. the pH content and the potential difference determined by the related ionoselective electrode 22.

Once analysis of the sample of the maximum calibrating element has been completed and said maximum pairs have been drawn, the command and control unit 70 operates ejection of the sample of maximum calibrating element that is sent into one of the vats 31 adapted to collect rejects or wastes.

When discharge has been completed, the second calibration is carried out that, utilising the minimum calibrating element, for each physico-chemical features enables a minimum pair to be determined which is formed of the related first parameter and the related value obtained with the minimum calibrating element. When said two calibrating analyses have been completed, the command and control unit 70 for each physico-chemical feature carries out a linearisation of the values included between the maximum pair and the minimum pair. By the term "linearisation" it is identified the fact that the command and control unit 70 assigns a linear development between the maximum and minimum pairs, i.e. assigns a constant development/variation of the value of the physico-chemical feature, preferably on a logarithmic base, relative to the first parameter.

In particular, the command and control unit 70 for each physico-chemical feature carries out a linearisation on a logarithmic scale, i.e. a linearisation between the first parameter and the logarithm of the physico-chemical feature value.

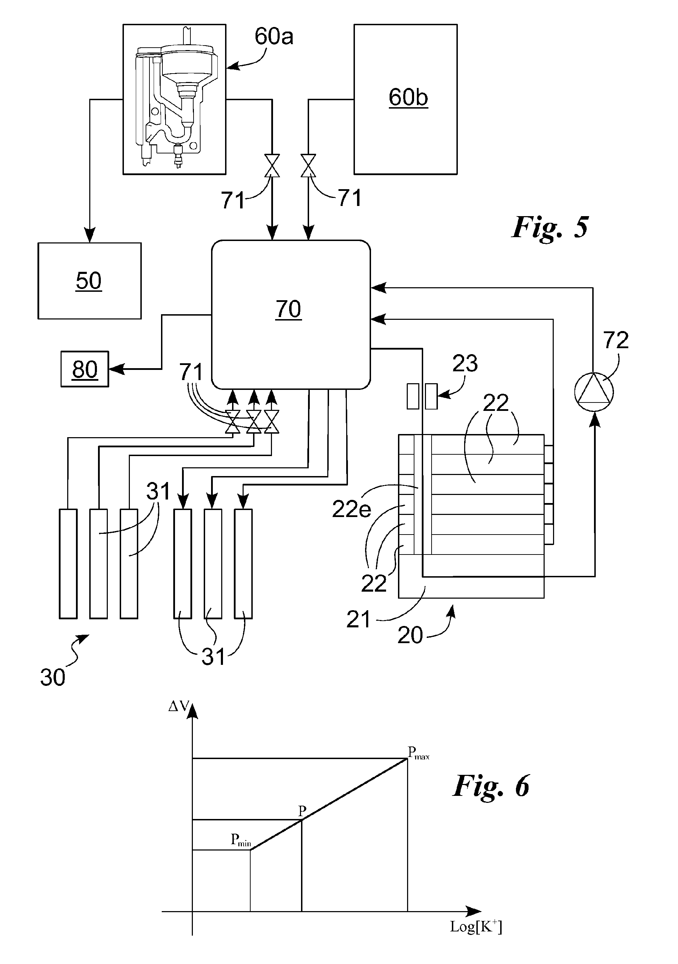

An example of this linearisation is reproduced in the graph in Fig. 6. In said graph on the x-axis there is the logarithm of the value of the physico-chemical feature under examination and on the y-axis the value of the first parameter, i.e. the potential difference between the electrodes. Therefore reproduced in the graph are the Pₘₐₓ and Pₘᵢₙ points identifying the maximum pair and minimum pair, respectively.

Once linearisation has been completed, the analysing device 1 is calibrated and ready for the analysis and, through the governing post 80, the operator can select the physico-chemical features that are wished to be analysed and start the analysing operation.

The command and control unit 70 gives a command for entry of the fluid to be analysed, i.e. urine, inside the duct consisting of the holding chambers 22e and carries out the sample urinalysis to a temperature of about 37°C, suitably checked by thermostat 23.

At the beginning, for each physico-chemical feature the first parameter is determined, i.e. the potential difference between the reference electrode 21 and the ionoselective electrode 22.

Subsequently, the command and control unit 70 calculates the individual physico-chemical features through a comparison between the first parameters obtained from the analysis of said sample and the aforesaid linearisation.

In particular, for each physico-chemical feature, unit 70 reproduces a first parameter relating to a feature on the respective linearisation and, based on the latter, draws the corresponding value of the physico-chemical feature in question.

For instance, if pH has to be calculated, the electrochemical sensor corresponding to pH detects the potential difference between the electrodes 21 and 22, i.e. the first pH parameter. This first parameter is then processed by the command and control unit 70 that reproduces the parameter on the graph (see Fig. 7) obtained in the linearisation, draws the pH pair indicated on the graph as point P and then obtains the pH value.

Once the analysis has been completed, the results are displayed on screen 91, printed and/or stored in suitable mass peripherals.

The command and control unit 70 operates ejection from the analysis station 20 of the fluid to be analysed, i.e. urine, that is collected in one of vats 31. Once vats 31 are exhausted, i.e. when vats 31 containing calibrating elements or washing fluid have exhausted their content, and/or vats 31 adapted to collect the waste fluids have been filled, block 30 is removed.

In particular, the calibrating/washing block 30 is pulled upwards producing a force that, winning opposition of the spring elements, causes movement of levers 43c locking plate 42 to the safety position.

Once block 30 has been moved apart, levers 43c pushed by the spring elements 43d go back to the starting position and device 1 is ready for introduction of a new block.

Alternatively, if only some of bags 31 are exhausted, it is possible to remove only those bags leaving block 30 stationary.

The invention further comprises a new process for urinalysis which is adapted to be preferably performed using the above described analysing device 1.

At the beginning the process contemplates a calibrating stage in which, utilising the maximum and minimum calibrating elements as described above, device 1 is calibrated for analysis.

This stage contemplates a step of first analysis in which the first parameters of each physical feature are determined. In detail, in this stage preferably determined are the potential differences between electrodes 21 and 22 as regards each feature.

The calibrating stage then contemplates a maximum calibrating step and a minimum calibrating step in which for each feature under examination a maximum pair and a minimum pair are obtained. In detail, each pair identifies the value of this feature as a function of a first parameter that, in case of electro-chemical analyses, consists of the potential difference between the reference electrode 21 and the ionoselective electrode 22.

These maximum and minimum pairs consist of the first parameter obtained in the aforesaid step and of the maximum value, obtained through said interpolation. In particular, the pairs are calculated taking into account not only the first parameter corresponding to the feature in question, but also the first parameters and/or values relating to at least another feature that could affect and therefore alter the analysis results.

Therefore in these stages the maximum and minimum pairs at least of the pH and the sodium, potassium, ammonium and chloride contents are determined by interpolating the various first parameters into each other. In detail, it is determined the value of the sodium content based on the first parameter relating to the sodium content and the first parameter relating to pH, while the value of the ammonium content is determined based on the first parameter relating to the ammonium content and the first parameter relating to the potassium content.

When said two calibration steps have been completed, this stage is finished with the linearisation step in which the values included between the maximum pair and the minimum pair are linearised, as above described.

Subsequently, an admission stage is provided in which for instance through the admission member 60b, a predetermined amount of urine is introduced into the device. Alternatively, this admission stage can be performed either in parallel or prior to the calibrating stage.

Once the sample has been introduced and device 1 has been calibrated, the analysis stage is started which comprises two sub-steps.

In the first analysis sub-step the electrochemical analysis of the sample is performed and for each physico-chemical feature to be analysed it is determined the first parameter, i.e. the potential differences between the reference electrode 21 and the ionoselective electrode 22.

When these potentials have been identified, the first analysis sub-step is finished and the second analysis sub-step starts, in which the value of each feature is determined by comparing the first parameters obtained in the first analysis sub-step with the result of said linearisation step.

Finally, the process can contemplate a plurality of said analysis stages performed at predetermined time intervals so as to enable the patient's state to be monitored in a substantially continuous manner.

The invention enables important advantages to be achieved.

In fact, the device allows analyses to be made on samples of urine exactly as it is, which analyses, taking place without the aid of external operators as said samples are not poured from a vessel to another, are devoid of polluting agents. In detail, the advantageous direct connection between patient and device 1, adapted to enable the fluid to directly reach the analysing device 1, allows the urine inside the analysing device to be exactly as it is, i.e. substantially coincident with and equal to that produced by the patient and therefore devoid of the reactants and/or chemical solutions normally used. Consequently, device 1 substantially allows the kidneys' activity of the human body to be monitored and consequently monitoring of the main organs of the human body, i.e. heart, lungs and kidneys, to be completed as previously mentioned.

Another advantage is obtained due to the presence of the particular sampler 60a and, more specifically the innovative collecting trap 64 enabling continuous renewal of the fluid to be analysed. In particular, this trap 64 enables an urine sample corresponding to the latest urine produced by the patient to be always available for analysis.

In fact, a possible entry of urine into sampler 60a determines replacement of the urine sample previously housed in the first duct by one coming from the fluid that has just reached the sampler.

In addition, through the optical counter 63, it is possible to determine when urine reaches the collecting trap 64 and therefore to carry out an analysis on an urine sample produced by the patient immediately before.

Another advantage is given by the particular procedure for analysis/evaluation of the results and more specifically by the innovative interpolation procedure enabling elimination of the disturbances due to the presence of substances altering the evaluation of a given feature.

A further advantage is given by the optical counter that is simple, cheap and characterised by a high accuracy. This advantage is substantially obtained due to the interruption in reading the continuous signal for a predetermined non-reading time.

In detail, due to this non-reading time, possible sudden changes, reflections or other disturbances that are created as a result of the interruption of the continuous signal are prevented from giving rise to a wrong evaluation of the flow.

A further advantage is obtained due to the presence of thermostat 23 that, by keeping the temperature substantially constant, allows sudden temperature changes that can modify the analysis results to be avoided.

In addition, the possibility of having a temperature almost equal to 37°C allows the urinalysis to be carried out on an urine sample substantially having the same temperature as the human body so that values of the physico-chemical features corresponding to those at the inside of the patient are obtained. Another advantage resides in that the analysing device 1 is able to monitor the main renal values and the related urinary flows in a critical patient frequently and in an automatic manner.

Another objective of importance reached with this device is represented by the possibility of monitoring the state of the renal function in a critical patient through frequent measurements of pH and electrolytes and flow on urine samples just produced by said critical patient.

In fact, as device 1 can operate in an automatic manner, it does not require the continuous presence of an operator for working and therefore it is able to carry out analyses at suitably predetermined intervals through the governing post 80.

In addition, the analysing device 1, due to the presence of the optical counter 63, enables the urine production to be monitored, so that the urine volume produced by the patient can be exactly determined.

A further advantage of device 1 is in fact represented by the particular simplicity of the optical counter 63 that, by virtue of its particular method of working described above enables very precise measurements to be obtained.

## Claims

1. An analysing device (1) for urine adapted to analyse an urine sample exactly as it is and to determine the value of physico-chemical features of said urine sample; said analysing device (1) comprising a fluid admission system including a sampler (60a) adapted to be brought into connection for fluid passage with the patient and measure a fluid portion to be analysed; an analysis station (20) brought into connection for fluid passage with said fluid admission system and adapted to carry out said analysis of at least part of said fluid portion to be analysed; a command and control unit (70) adapted to analyse data collected by said analysis station (20); and a holding structure (40) adapted to support the components of said analysing device (1) in a correct position; and **characterised in that** said analysis station (20) comprises a plurality of ionoselective electrodes (22) each of which is adapted to determine a first parameter, said first parameter being a function of at least one of said values of said physico-chemical features, and **in that** said command and control unit (70) is adapted to carry out an interpolation between said first parameters for determining at least one of said values of said physico-chemical features.

2. An analysing device (1) as claimed in claim 1, comprising a thermostat (23) adapted to adjust the temperature of said urine to be analysed in at least said analysis station.

3. An analysing device (1) as claimed in one or more of the preceding claims, comprising a calibrating/washing block (30) including a plurality of vats (31) each of which is adapted to be put into connection for fluid passage with said analysis station (20); and wherein two of said collecting vats each contain a maximum calibrating element and a minimum calibrating element.

4. An analysing device (1) as claimed in the preceding claim, wherein, through said interpolation, said command and control unit (70) is adapted to determine, for each of said physico-chemical features, a maximum pair which is obtained through analysis of said maximum calibrating element and consists of the first maximum parameter and the maximum value obtained through said interpolation, and a minimum pair which is obtained through analysis of said minimum calibrating element and consists of the corresponding first minimum parameter and the minimum value obtained through said interpolation.

5. An analysing device (1) as claimed in one or more of claims 3-4, wherein said holding structure (40) comprises insertion means (41), each of which is adapted to be introduced into one of said collecting vats (31) and a lifting mechanism adapted to operate introduction of said insertion means (41) into said collecting vats (31) when said collecting vats (31) are inserted in said holding structure (40).

6. An analysing device (1) as claimed in one or more of the preceding claims, wherein said sampler (60a) comprises a dripping device (62) adapted to divide the urine coming from the patient into drops and a continuous-signal optical counter (63) adapted to count the drops coming out of said dripping device (62).

7. An analysing device (1) as claimed in the preceding claim, wherein said command and control unit (70) is adapted to stop reading of said continuous signal when said drop has intercepted said continuous signal for a predetermined non-reading time.

8. An analysing device (1) as claimed in the preceding claim, wherein said sampler (60a) comprises a collecting trap (64) placed downstream of said dripping device (62) and adapted to collect a predetermined amount of urine; and wherein said collecting trap (64) has a first duct (64a) having an S-shaped extension line and having the ends in connection for fluid passage with said dripping device and with a discharge tank, and wherein said analysing device (1) comprises a pipe adapted to bring the lower portion of said first duct (64a) into connection for fluid passage with said analysis station (20).

9. An analysing device (1) as claimed in the preceding claim, wherein said collecting trap (64) comprises a second duct (64b) adapted to bring two sections of said first duct (64a) placed on opposite side relative to said pipe into connection with each other for air passage.

10. A process for urinalysis adapted to determine the value of physico-chemical features of an urine sample through an electrochemical analysis; said process comprising a calibrating stage adapted to calibrate an analysing device through a maximum calibrating element and a minimum calibrating element; and an analysis stage adapted to analyse an urine sample to be analysed inserted in said device; and **characterised in that** in said analysis stage the analysis takes place on urine exactly as it is.

11. A process for urinalysis as claimed in the preceding claim, wherein said calibrating stage comprises a step of first analysis in which the first parameters of said physico-chemical features are determined for said maximum calibrating element and minimum calibrating element; and a maximum calibrating element step and a minimum calibrating element step in which for each of said physico-chemical features a maximum pair relating to said maximum calibrating element and a minimum pair relating to said minimum calibrating element respectively are obtained, which are made up of the value of one of said physico-chemical features and said first parameter of said physico-chemical feature; and wherein said values of said physico-chemical features of said maximum pair and minimum pair are obtained through interpolation between said first parameters.

12. A process for urinalysis as claimed in the preceding claim, wherein in said calibrating stage the maximum pairs and minimum pairs are calculated at least of the pH and the sodium, potassium, ammonium and chloride contents, and wherein said value of said sodium content is determined based on said first parameter relating to said sodium content and said first parameter relating to said pH and said value of said ammonium content is calculated based on said first parameter relating to said ammonium content and said first parameter relating to said potassium content.

13. A process for urinalysis as claimed in claims 11-12, wherein, for each of the physico-chemical features, a linearisation is carried out in said calibrating stage in which the values included between said maximum pair and minimum pair are linearised.

14. A process for urinalysis as claimed in the preceding claim, comprising an analysis stage in which said first parameter of said physico-chemical features relating to said urine sample are determined, and wherein in said analysis stage said values of said features are determined by comparing said first parameters of said sample to be analysed with the results of said linearisation.

15. A process for urinalysis as claimed in claims 10-14, wherein at least said analysis stage is repeated at predetermined time intervals.
